# EUROPEAN PATENT APPLICATION

(11) **EP 4 840 961 A1**
(43) Date of publication of application: **18.11.2026**
(21) Application number: 24950361.6
(22) Date of filing: 10.10.2024
(51) Int. Cl.: C12N 15/70, C12N 15/53, C12N 15/54, C12N 15/31, C12N 1/21, C12P 13/04, C12R 1/19

(54) **GENETICALLY ENGINEERED BACTERIUM WITH HIGH YIELD OF D-PANTOTHENIC ACID BASED ON ONE-CARBON UNIT SUPPLY, CONSTRUCTION METHOD THEREFOR AND USE THEREOF**

(30) Priority: 06.08.2024 CN 202411070976
(71) Applicant: Zhejiang University of Technology, Hangzhou, Zhejiang 310014 (CN)
(72) Inventor: ZHANG, Bo, Hangzhou, Zhejiang 310014 (CN); WU, Jie, Hangzhou, Zhejiang 310014 (CN); LIU, Zhiqiang, Hangzhou, Zhejiang 310014 (CN); TANG, Mengna, Hangzhou, Zhejiang 310014 (CN); WANG, Guanghao, Hangzhou, Zhejiang 310014 (CN); ZHENG, Yuguo, Hangzhou, Zhejiang 310014 (CN)
(74) Representative: Fezzardi, Antonio
(86) International application number: PCT/CN2024/123802
(87) International publication number: WO 2026/031334

(57) **Abstract**

The present invention provides a genetically engineered bacterium for high-yield production of D-pantothenic acid based on one-carbon unit supply, a construction method thereof, and use of said bacterium in the preparation of D-pantothenic acid by microbial fermentation. In the present invention, the glycine cleavage system is enhanced to strengthen the supply of one-carbon cofactors in Escherichia coli, thereby directing carbon flux toward D-pantothenic acid synthesis. To further enhance the carbon flux, genes related to the tetrahydrofolate (THF) synthesis pathway are strengthened, the serine biosynthesis pathway is dynamically regulated, and expression of key genes in the pantoate biosynthesis pathway is enhanced in combination with enhancement of the glycine cleavage system. Ultimately, a plasmid-free, antibiotic-free engineered strain for high-yield production of D-pantothenic acid is obtained. Compared with the starting strain, the shake-flask titer of D-pantothenic acid is increased by 31.7%, reaching 6.85 g/L; and in a 5-L fermenter fed-batch fermentation for 92 h, the D-pantothenic acid yield can reach 112.62 g/L.

## Description

### TECHNICAL FIELD

The present invention belongs to the technical field of genetic engineering, and specifically relates to a genetically engineered bacterium for high-yield production of D-pantothenic acid based on one-carbon unit supply, a construction method thereof, and use of the genetically engineered bacterium for high-yield production of D-pantothenic acid in the preparation of D-pantothenic acid by microbial fermentation.

### BACKGROUND ART

Pantothenic acid, also known as vitamin B5, is one of the constituent components of coenzyme A, and plays a key role in important biochemical reactions such as energy metabolism and the citric acid cycle. Therefore, as an important vitamin and precursor substance, D-pantothenic acid is widely used in feed, medicine, cosmetics, and other fields. Among known methods for synthesizing D-pantothenic acid, the production of D-pantothenic acid by biological fermentation has attracted attention due to advantages such as inexpensive substrates, easy separation, and low toxicity. However, at present, the production of D-pantothenic acid by biological methods still has drawbacks, such as problems of unstable fermentation process and low yield. Therefore, constructing a D-pantothenic acid-producing strain with even higher yield remains a major challenge.

### SUMMARY OF THE INVENTION

In order to solve the technical problem in the prior art of low yield of D-pantothenic acid synthesized by microbial fermentation, the present invention uses CRISPR-Cas9-mediated gene editing technology to enhance the glycine cleavage system and the folate synthesis pathway in the genome of a chassis bacterium, regulate the serine pathway, and enhance the expression of key genes of the pantoate synthesis pathway, thereby obtaining a genetically engineered bacterium for high-yield production of D-pantothenic acid, and applying the genetically engineered bacterium to the preparation of D-pantothenic acid by microbial fermentation.

The technical solution adopted by the present invention is: a method for constructing a genetically engineered bacterium for high-yield production of D-pantothenic acid based on one-carbon unit supply, the method comprising: using CRISPR-Cas9-mediated gene editing technology, in the genome of a chassis bacterium: overexpressing the *gcvTHP* gene; enhancing the *folP* gene; integrating a *serA* and *glyA* gene cluster and adding one copy of the *panB* gene after the *serAglyA* gene cluster, wherein the *serAglyA* gene cluster is regulated by a dynamic regulatory element; and introducing a heterologous codon-optimized *alsS* gene, thereby obtaining a genetically engineered bacterium for high-yield production of D-pantothenic acid.

On the basis of the chassis bacterium DPAP2 (E.coli W3110, Trc-*EcilvD**/ *BspanBA**/ C*gpanC**/ *alsS**/ *BspanB**/ *nac^{GTG}* / ΔP_{345ptsH} / *gltA^{GTG}*/ *gltA^{TTG}*/ Trc-*PpfkB*, which has been disclosed in CN116590209A), the present invention comprehensively applies systems metabolic engineering strategies, uses CRISPR/Cas9 gene editing technology to replace the native promoter of the *gcvTHP* gene with the pTrc promoter, thereby eliminating the transcriptional repression of the *gcvTHP* gene by the *gcvA* and *purR* genes, enhances the glycine cleavage system, and strengthens the supply of one-carbon cofactors in the Escherichia colicell, further directing carbon flux toward the synthesis of D-pantothenic acid. On this basis, in order to further enhance carbon flux toward the synthesis of D-pantothenic acid, the present invention also strengthens genes related to the THF synthesis pathway. On one hand, by adding one copy of the *folP* gene at the *lafU* pseudogene locus to enhance folate synthesis, providing a methyl carrier for one-carbon units. On the other hand, using an Apt2#82 glycine riboswitch to control the expression of the *serAglyA* gene cluster in real time according to the intracellular glycine concentration, achieving moderate enhancement of key genes of the serine pathway, so as to reasonably regulate the serine synthesis rate. Meanwhile, in order to respond to the supply of methyl groups, the copy number of the *panB* gene regulated by the PpanB promoter is increased after the *serAglyA* gene cluster. Finally, by introducing a heterologous codon-optimized *alsS*, the expression efficiency of the genetically engineered bacterium is improved, and the yield of D-pantothenic acid is increased, ultimately obtaining a plasmid-free, antibiotic-free engineered strain for high-yield production of D-pantothenic acid.

**Table 1. Genes involved in gene editing and corresponding pathways**

| **Gene** | **Pathway involved** |
|---|---|
| *gcvTHP* | Glycine synthesis |
| *folP* | Folate synthesis |
| *serAglyA* | Glycine synthesis |
| *panB* | Pantoate synthesis |
| *alsS* | Pantoate synthesis |

Preferably, the method for overexpressing the *gcvTHP* gene comprises: replacing the native promoter of the *gcvTHP* gene with a pTrc promoter. Activating the glycine cleavage system through a strong promoter can eliminate the influence of *gcvA* and *purR* on the supply of one-carbon units, provide 5,10-methylenetetrahydrofolate for the D-pantoate pathway, and effectively increase the synthesis of D-pantothenic acid.

Preferably, the method for enhancing the *folP* gene comprises: adding one copy of the *folP* gene at the lafU pseudogene locus. Overexpressing the *folP* gene can further promote the combination of pABA and the GTP pathway, and is an effective strategy for strengthening folate synthesis to provide a methyl carrier for one-carbon units.

Preferably, the dynamic regulatory element is a riboswitch Apt2#82. The riboswitch Apt2#82 can control the overexpression of *serA* and *glyA* according to the intracellular glycine concentration. When glycine accumulates to a certain level, it folds to cover the ribosome binding site, repressing the translation of the *serAglyA* gene cluster; when the intracellular glycine content is low, it exposes the ribosome binding site to enhance translation. Using the riboswitch to control the expression of *glyA* in real time can achieve moderate enhancement of key genes of the serine pathway. This strategy can reasonably regulate the serine synthesis rate, maintain the intracellular glycine at a low level, and effectively promote the synthesis of D-pantothenic acid.

Preferably, the *panB* gene is regulated by the PpanB promoter. Adding one more copy of the *panB* gene after the *serAglyA* gene cluster can better respond to the supply of methyl groups and promote the synthesis of D-pantothenic acid.

Preferably, the *alsS* gene is derived from *Bacillus subtilis* and is codon-optimized. Acetolactate synthase (*alsS*) catalyzes the formation of α-acetolactate from two molecules of pyruvate, and acetolactate synthase is involved in the synthesis of valine, leucine, and isoleucine. By introducing a heterologous *alsS* gene into the genome and performing codon optimization, the expression efficiency of the gene in the host can be improved, and the burden on the strain is relatively small, promoting an increase in D-pantothenic acid yield in shake flasks without the addition of pyruvate.

Preferably, the chassis bacterium is: E. coliW3110, Trc-*EcilvD**/*BspanBA**/*CgpanC**/ *alsS**/*BspanB**/*nac^{GTG}* /ΔP_{345ptsH}/*gltA^{GTG}* /*gltA^{TTG}* /Trc-*PpfkB*, designated as DPAH2.

Preferably, the nucleotide sequences of the *gcvTHP* gene, the *folP* gene, and the *alsS* gene regulated by the pTrc promoter are shown as SEQ ID NO. 1, SEQ ID NO. 2, and SEQ ID NO. 3, respectively; the sequence of the *serAglyA* gene cluster regulated by the Apt2#82 riboswitch is shown as SEQ ID NO. 4; the nucleotide sequence of the *panB* gene regulated by the PpanB promoter is shown as SEQ ID NO. 5.

More specifically, the method for constructing the genetically engineered bacterium for high-yield production of D-pantothenic acid comprises the following steps:
(1) Using the engineered bacterium DPAP2 as the starting strain and designating it as H2, using CRISPR-Cas9-mediated gene editing technology, replacing the native promoter of the *gcvTHP* gene with a Trc promoter in its genome, deleting the transcriptional regulatory region, to obtain an engineered bacterium DPAH3 derivative, Trc-*gcvTHP*, designated as engineered bacterium DPAH3;
(2) Using the engineered bacterium DPAH3 as the starting strain, using CRISPR-Cas9-mediated gene editing technology, adding one copy of the *folP* gene at the *lafU* pseudogene locus, to obtain an engineered bacterium DPAH6 derivative, *lafU*::*folP*, designated as engineered bacterium DPAH6;
(3) Using the engineered bacterium DPAH6 as the starting strain, using CRISPR/Cas9 gene editing technology, integrating a serA and *glyA* gene cluster into the genome, and adding one copy of the *panB* gene after the gene cluster, to obtain an engineered bacterium DPAH7 derivative, yeeP::Trc-Apt2#82-*serAglyA*-PpanB-*panB*, designated as engineered bacterium DPAH7;
(4) Using the engineered bacterium DPAH7 as the starting strain, using CRISPR/Cas9 gene editing technology, increasing the copy number of the *Bacillus subtilis alsS* gene regulated by the pTrc promoter in the genome by means of gene knock-in, performing codon optimization, to obtain an engineered bacterium DPAH8 derivative, *gapC*::Trc-*alsS**, designated as engineered bacterium DPAH8, which is the genetically engineered bacterium for high-yield production of D-pantothenic acid.

The present invention also provides a genetically engineered bacterium for high-yield production of D-pantothenic acid constructed by any one of the above methods. By the above methods, the present invention constructs an engineered bacterium that is plasmid-free and free of antibiotic addition during fermentation: E. coliW3110, Trc-*EcilvD**/ *BspanBA**/ *CgpanC**/ *alsS**/ *BspanB**/ *nac^{GTG}*/ ΔP_{345ptsH}/ *gltA^{GTG}*/ *gltA^{TTG}*/ Trc-*PpfkB*/ Trc-*gcvTHP*/ *lafU*::*folP*/ yeeP::Trc-Apt2#82-*serAglyA*-PpanB-*panB*/ *gapC*::Trc-*alsS**, the shake-flask titer of this strain reaches 6.85 g/L, which is increased by 31.7% compared to the starting strain, and finally, using 5-L fermenter fed-batch fermentation for 92 hours, the D-pantothenic acid yield can reach 112.6 g/L, greatly improving the synthesis of D-pantothenic acid and shortening the fermentation period.

The present invention also provides use of the genetically engineered bacterium for high-yield production of D-pantothenic acid in the preparation of D-pantothenic acid by microbial fermentation.

Preferably, the method of the use comprises: inoculating the genetically engineered bacterium for high-yield production of D-pantothenic acid into a fermentation medium, performing fed-batch fermentation culture at 28~37°C, 300~450 rpm, pH 6.7~6.9, and dissolved oxygen of 10~30% for 72~96 h, and after fermentation is completed, separating and purifying to obtain the D-pantothenic acid.

Preferably, the fermentation medium has the following composition: glucose 10~30 g/L, ammonium sulfate 10~25 g/L, anhydrous betaine 1~5 g/L, yeast extract 1~5 g/L, potassium dihydrogen phosphate 1~5 g/L, anhydrous magnesium sulfate 0.5~2 g/L, β-alanine 1~5 g/L, 1~5 mL/L of a trace element solution, with deionized water as solvent, and natural pH; the trace element solution has the following composition: 10 g/L CuCl₂, 10 g/L FeSO₄·7H2O, 10 g/L ZnSO₄·7H₂O, 0.2 g/L CuSO₄, 0.02 g/L NiCl₂·7H₂O, with deionized water as solvent.

Preferably, the method of the use comprises: loading 1~3 L of fermentation medium into a 5 L fermenter, sterilizing at 115°C for 30 min, inoculating the genetically engineered bacterium strain into the 1~3 L of fermentation medium, performing fermentation culture at 28~37°C, with an initial aeration rate of 3~6 L/min and an initial stirring speed of 300~450 rpm, adjusting pH with aqueous ammonia, while adding IPTG at a final concentration of 0.1~0.4 mM, VB₁ at a final concentration of 5 mg/L, VB₁₂ at a final concentration of 2 mg/L, and 5~10 mL of isoleucine at 10~40 g/L; during the fermentation process, maintaining dissolved oxygen at 10~30% using DO-cascade stirring, maintaining pH at 6.7~6.9 using aqueous ammonia as a neutralizing agent, adding a feed medium into the tank via pH-stat feeding, controlling the glucose concentration below 5 g/L, culturing at 28~37°C for 72~96 hours to obtain a fermentation broth, and taking the supernatant of the fermentation broth for separation and purification to obtain the D-pantothenic acid. Wherein, the feed medium has the following composition: glucose 500 g/L, ammonium sulfate 5~25 g/L, anhydrous betaine 2~8 g/L, yeast extract 1~5 g/L, potassium dihydrogen phosphate 10~20 g/L, anhydrous magnesium sulfate 5~15 g/L, β-alanine 40~100 g/L, 1~5 mL/L of a trace element solution, with deionized water as solvent, and natural pH.

Beneficial Effects of the Present Invention: The present invention uses CRISPR/Cas9 gene editing technology to enhance the glycine cleavage system and the folate synthesis pathway on the basis of an existing engineered bacterium, providing one-carbon units and enriching methyl carriers for the pantoate synthesis pathway. On the other hand, an Apt2#82 glycine riboswitch is used to finely control the synthesis rates of serine and glycine, and the expression levels of key genes in the D-pantoate biosynthesis pathway are further enhanced, continuously supplying methyl groups for the entire reaction system. A high-yield strain that is plasmid-free and does not require antibiotic addition during fermentation is obtained; the shake-flask titer of the strain of the present invention reaches 6.85 g/L, which is increased by 31.7% compared to the starting strain, and finally, using 5 L fermenter fermentation for 92 hours, the D-pantothenic acid yield can reach 112.6 g/L, greatly improving the synthesis of D-pantothenic acid and shortening the fermentation period.

### BRIEF DESCRIPTION OF THE DRAWINGS

FIG. 1 is a diagram of the D-pantothenic acid metabolic pathway and modification sites.
FIG. 2 shows the changes in OD₆₀₀ and D-pantothenic acid titer of DPAH3 in Example 1.
FIG. 3 shows the changes in OD₆₀₀ and D-pantothenic acid titer of DPAH6 in Example 2.
FIG. 4 shows the changes in OD₆₀₀ and D-pantothenic acid titer of DPAH7 in Example 3.
FIG. 5 shows the changes in OD₆₀₀ and D-pantothenic acid titer of DPAH8 in Example 4.
FIG. 6 is a graph showing the fermentation results of DPAH8 in a 5 L bioreactor in Example 5.

### DETAILED DESCRIPTION OF EMBODIMENTS

The embodiments of the present invention are described below through specific examples. A person skilled in the art can readily understand other advantages and effects of the present invention from the disclosure of this specification. The present invention may also be practiced or applied in other different specific embodiments; various details in this specification may also be modified or altered based on different viewpoints and applications without departing from the spirit of the present invention. It should be noted that, where there is no conflict, the following examples and features in the examples may be combined with each other. Unless otherwise specified, all methods used in the embodiments of the present invention are conventional methods, and all reagents used are commercially available.

In the following examples, the final concentration of spectinomycin in the culture medium is 0.05 mg/L, and the final concentration of kanamycin in the culture medium is 0.05 mg/L.

The parent strain E.coli W3110 of the present invention is from the Coli Genetic Stock Center (CGSC) of Yale University, with a deposit date of August 5, 1975, and a deposit number of CGSC#4474, and has been disclosed in patents US 2009/0298135 A1 and US 2010/0248311 A1.

The HPLC determination method for D-pantothenic acid content is as follows: Chromatographic conditions: C18 column (250 × 4.6 mm, particle size 5 µm, Agilent Technologies Co., Santa Clara, CA, USA), detection wavelength: 200 nm, column temperature: 30°C; sample treatment: dilute the sample with ultrapure water, keeping the D-pantothenic acid content between 0.05 g/L and 0.40 g/L; mobile phase: acetonitrile/water/phosphoric acid (50/949/1); data acquisition time: 25 min.

### Example 1: Construction of DPAH3 and Shake-Flask Fermentation

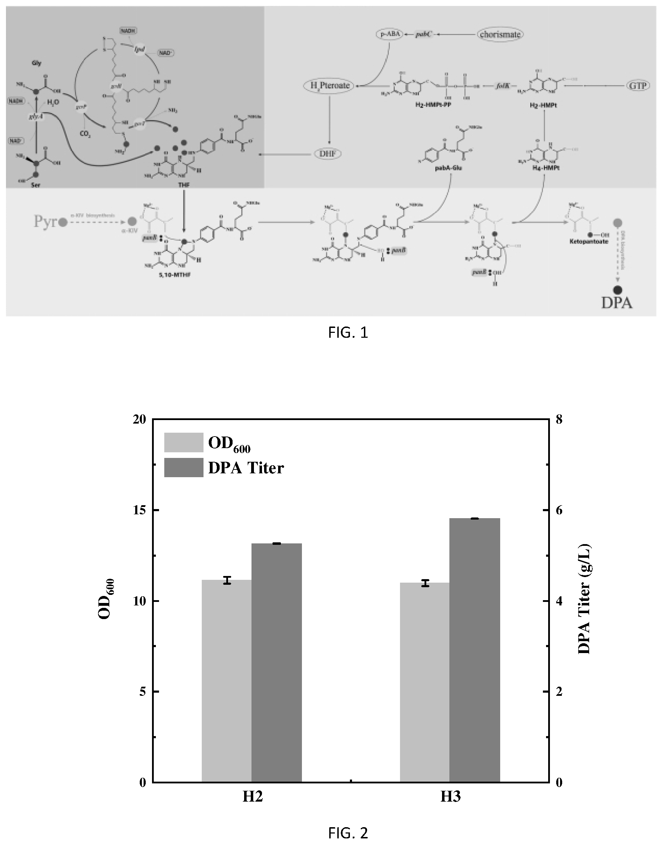

Using DPAP2 (E.coli W3110, Trc-*EcilvD**/ *BspanBA**/ *CgpanC**/ *alsS**/ *BspanB**/ *nac^{GTG}*/ ΔP_{345ptsH}/ *gltA^{GTG}*/ *gltA^{TTG}*/ Trc-*PpfkB*) as the starting strain, CRISPR-Cas9-mediated gene editing technology is used to overexpress *gcvTHP* in the genome, and replace the native glycine promoter with a Trc promoter (nucleotide sequence shown as SEQ ID NO. 1), so that the transcriptional regulatory factors *gcvA* and *purR* cannot inhibit the expression of the glycine cleavage system, thereby obtaining strain DPAH3 (DPAH2 derivative, Trc-gcvTHP).
(1) Construction of the pTarget-PAM-*gcvTHP* plasmid: Using the pTarget plasmid (Addgene Plasmid #62226) as a template and pT-*gcvTHP*-PAM-F/R as primers, PCR amplification is performed. After the PCR product is verified by nucleic acid gel electrophoresis, it is digested with *Dpn*I restriction enzyme at 37°C for 3 h, and then transformed into E.coli DH5α. After screening on spectinomycin plates and sequencing verification, the correct pTarget-PAM-*gcvTHP* plasmid is obtained for subsequent ligation with Donor DNA.
(2) Construction of the pTD-donor-*EcgcvTHP* plasmid: First, using the E.coli W3110 genome as a template and *gcvTHP*-up-F/R as primers, the upstream portion (F1) of the donor DNA is amplified, and using *gcvTHP*-down-F/R as primers, the downstream portion (F2) of the donor DNA is amplified. Then, using the E.coli W3110 genome as a template and pTrc-*EcgcvTHP*-18F/R as primers, the *EcgcvTHP* gene fragment carrying the pTrc promoter (F3) is amplified. The PCR fragments F1, F2, and F3 are purified by gel extraction. The pTarget-*gcvTHP* plasmid is digested with XbaI and PstI at 37°C for 8 h, and the DNA fragment is recovered using a Clean up kit. According to the instructions of ClonExpress^{®} (One step clone kit, Vazyme Biotech, Nanjing, China), the pTarget-*gcvTHP* vector and fragments F1, F2, and F3 are ligated together, and the pTD-donor-*EcgcvTHP* plasmid is obtained through sequencing verification.
(3) The pCas plasmid (Addgene Plasmid #62225) is introduced into ZJUTDPAH2 (E.coliW 3110, Trc-*EcilvD**/ *BspanBA**/ *CgpanC**/ *alsS**/ *BspanB**/ *nac^{GTG}*/ ΔP_{345ptsH} / *gltA^{GTG}*/ *gltA^{TTG}*/ Trc-*PpfkB*). A single monoclonal colony is transferred to an LB test tube containing 0.05 mg/L kanamycin and cultured overnight at 30°C. Then, it is inoculated at a volume concentration of 1% into a 250 mL shake flask containing 50 mL of LB medium, and 500 µL of 1 mol/L L-arabinose is added, and cultured at 150 rpm and 30°C until OD₆₀₀ reaches 0.4~0.6. Cells are collected by centrifugation at 4000 rpm and 4°C for 10 min to prepare electrocompetent cells. The detailed process is described in (Molecular Cloning: A Laboratory Manual, 3rd Edition, pp. 99-102).
(4) An appropriate amount (approximately 200 ng) of the pTD-donor-*EcgcvTHP* plasmid is taken with a pipette and mixed with 100 µL of pre-prepared electrocompetent cells. The mixture is transferred into a pre-cooled 2 mm electroporation cuvette, incubated on ice for about 1~2 min, and then electroporation transformation is performed using an electroporator (MicroPulser^{™}, BIO-RAD). Immediately after electroporation, 800 µL of LB medium is added, gently aspirated immediately, and transferred to a 2 mL EP tube. After recovery at 30°C for 3~4 h, the culture is spread onto an LB solid plate containing 0.05 mg/L kanamycin and 0.05 mg/L spectinomycin, and incubated upside down at 30°C for 12~16 h. Colony PCR verification is performed using *gcvTHP*-VF/R as primers. If a fragment of approximately 3500 bp is successfully cloned, it confirms a positive colony of DPAH3 (DPAH2 derivative, Trc-*gcvTHP*).
(5) Plasmid elimination: A positive single colony is picked with an inoculation loop and inoculated into an LB liquid test tube containing 1 mM IPTG and 0.05 mg/L kanamycin, and cultured overnight at 30°C. The next day, the bacterial culture is streaked onto an LB solid plate containing 0.05 mg/L kanamycin, and cultured at 30°C for 24 h. When the colonies have grown to a certain size, some single colonies are picked and streaked onto an LB plate containing 0.05 mg/L spectinomycin. Single colonies that cannot grow on the LB plate containing 0.05 mg/L spectinomycin indicate that their pTarget-*gcvTHP* plasmid has been successfully eliminated. Thereafter, single colonies with successfully eliminated pTarget-*gcvTHP* plasmid are picked into an LB test tube and cultured overnight at 37°C for elimination of the pCas plasmid. The next day, the bacterial culture is streaked onto an LB plate, and after culturing at 37°C for 12 h, some single colonies are picked and streaked onto an LB plate containing 0.05 mg/L kanamycin. Single colonies that cannot grow on the LB plate containing 0.05 mg/L kanamycin indicate that their pCas plasmid has been successfully eliminated, finally obtaining a plasmid-free strain. Using the starting strain H2 as a control group, both are inoculated into 10 mL of LB medium and cultured at 37°C and 200 rpm as precultures. After 8~12 h, 1 mL of the preculture is inoculated at a 2% inoculum size into a 500 mL shake flask containing 50 mL of MS medium, and then cultured in a constant-temperature shaker at 30°C and 180 rpm for 48 h for strain fermentation. After fermentation is completed, 1 mL of fermentation broth is taken to measure the OD₆₀₀ value. At the same time, 1 mL of fermentation broth is taken with a pipette, centrifuged at 12000 rpm and room temperature for 3 min, the fermentation supernatant is diluted 5-fold, and the diluted sample is subjected to impurity removal treatment using an aqueous filter membrane, followed by HPLC detection. The OD₆₀₀ and the D-pantothenic acid content in the fermentation supernatant are shown in FIG. 2.

As can be seen from the figure, after additionally increasing the copy number of the *gcvTHP* gene in the genome, the D-pantothenic acid yield of DPAH3 after shake-flask fermentation for 48 h increased by 11.7%, reaching 5.81 g/L. By activating the glycine cleavage system through a strong promoter and eliminating the influence of *gcvA* and *purR* on the supply of one-carbon units, 5,10-methylenetetrahydrofolate is provided for the D-pantoate pathway, thereby successfully increasing the D-pantothenic acid yield.

LB medium: 10 g/L tryptone, 5 g/L yeast extract, 5 g/L NaCl, with deionized water as solvent, natural pH.

MS medium: glucose 20 g/L, (NH₄)₂SO₄ 16 g/L, KH₂PO₄ 2 g/L, MgSO₄ 0.5 g/L, yeast extract 2 g/L, CaCO₃ 10 g/L, 1 mL/L trace element solution, with deionized water as solvent, natural pH; 10 g/L calcium carbonate (sterilized separately); the trace element solution has the following composition: 10 g/L CuCl₂, 10 g/L FeSO₄·7H₂O, 1 g/L ZnSO₄·7H₂O, 0.20 g/L CuSO₄, 0.02 g/L NiCl₂·7H₂O, with deionized water as solvent.

### Example 2: Construction of DPAH6 and Shake-Flask Fermentation

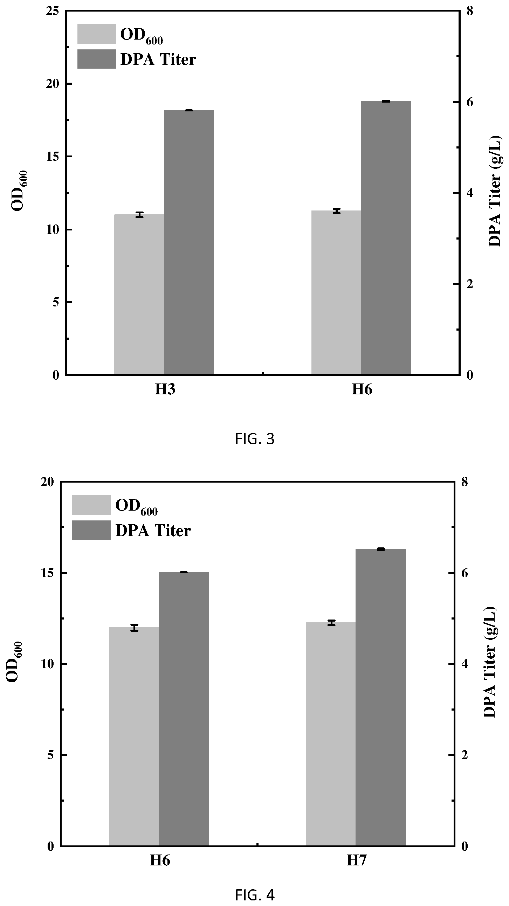

Using DPAH3 as the starting strain, CRISPR-Cas9-mediated gene editing technology is employed, and by means of gene knock-in, one copy of the *folP* gene (nucleotide sequence shown as SEQ ID NO. 2) is added at the *lafU* pseudogene locus, thereby obtaining DPAH6 (derivative, *lafU*::*folP*).
(1) Construction of the pTarget-PAM-*folP* plasmid: Using the pTarget plasmid (Addgene Plasmid #62226) as a template and pT-*folP*-PAM-F/R as primers, PCR amplification is performed. After the PCR product is verified by nucleic acid gel electrophoresis, it is digested with Dpn I restriction enzyme at 37°C for 3 h, and then transformed into E.coli DH5α. After screening on spectinomycin plates and sequencing verification, the correct pTarget-PAM-*folP* plasmid is obtained for subsequent ligation with Donor DNA.
(2) Construction of the pTD-Donor-*EcfolP* plasmid: First, using the E.coli W3110 genome as a template and *folP*-up-F/R as primers, the upstream portion (F1) of the donor DNA is amplified, and using *folP*-down-F/R as primers, the downstream portion (F2) of the donor DNA is amplified. Then, using the E. coli W3110 genome as a template and pTrc-*EcfolP*-F/R as primers, the *EcfolP* gene fragment carrying the pTrc promoter (F3) is amplified. The PCR fragments F1, F2, and F3 are purified by gel extraction. The pTarget-*folP* plasmid is digested with XbaI and PstI at 37°C for 8 h, and the DNA fragment is recovered using a Clean up kit. According to the instructions of ClonExpress^{®} (One step clone kit, Vazyme Biotech, Nanjing, China), the pTarget-*ydeU* vector and fragments F1, F2, and F3 are ligated together, and the pTD-*EcfolP* plasmid is obtained through sequencing verification.
(3) The pCas plasmid (Addgene Plasmid #62225) is introduced into the competent cells of DPAH3 obtained in Example 1. The method for preparing electrocompetent cells of DPAH3 is the same as that in Example 1 (3).
(4) Positive colonies of DPAH6 are constructed, and the construction method is the same as that in Example 1 (4).
(5) Plasmid elimination: The implementation method is the same as that in Example 1 (5), obtaining plasmid-free DPAH6 (derivative, *lafU*::*folP*).
(6) The constructed DPAH6 production strain is subjected to shake-flask testing and detection using DPAH3 constructed in Example 1 as a control group. The OD₆₀₀ and the D-pantothenic acid content in the fermentation supernatant are shown in FIG. 3.

As can be seen from the figure, by adding one copy of the *folP* gene at the *lafU* pseudogene locus in DPAH3, the D-pantothenic acid yield of DPAH6 was increased to 6.01 g/L. Therefore, overexpressing *folP* promotes the combination of pABA and the GTP pathway, and is an effective strategy for strengthening folate synthesis to provide a methyl carrier for one-carbon units.

### Example 3: Construction of DPAH7 and Shake-Flask Fermentation

Using DPAH6 as the starting strain, CRISPR/Cas9 gene editing technology is employed to utilize a pseudogene locus in the genome of strain DPAH6, integrate a *serA* and *glyA* gene cluster into the genome, and design a dynamic regulatory element to adjust the expression state of the gene cluster in real time. In order to respond to the supply of methyl groups, we add one more copy of the *panB* gene after the gene cluster, wherein the gene is controlled by the native PpanB promoter (nucleotide sequences shown as SEQ ID NO. 4 and SEQ ID NO. 5), thereby obtaining DPAH7 (DPAH6 derivative, yeeP::Trc-RBSApt2#82-*serAglyApanB*).
(1) Construction of the pTarget-PAM-*serAglyApanB* plasmid: Using the pTarget plasmid (Addgene Plasmid #62226) as a template and pT-PAM-*serAglyApanB*-F/R as primers, PCR amplification is performed. The PCR product is digested with Dpn I at 37°C for 3 h. The PCR product of pTarget-PAM-*serAglyApanB* is amplified using linearization primers pTarget-XF and pTarget-XR, and the linearized pTarget-PAM-*serAglyA* vector is purified by gel extraction for subsequent ligation with Donor DNA.
(2) Construction of the pTD-Donor-*serAglyApanB* plasmid: Using the E.coli W3110 genome as a template and *serAglyApanB*-up-F and *serAglyApanB*-up-R as primers, the upstream portion (F1) of the donor DNA is amplified, and using *serAglyApanB*-down-F and *serAglyApanB*-down-R as primers, the downstream portion (F2) of the donor DNA is amplified. The PCR fragments F1 and F2 are purified by gel extraction. According to the instructions of ClonExpress^{®} (One step clone kit, Vazyme Biotech, Nanjing, China), the linearized pTarget-*serAglyApanB* vector and fragments F1 and F2 are ligated together, and the pTD-*serAglyApanB* plasmid is obtained through sequencing verification.
(3) The pCas plasmid (Addgene Plasmid #62225) is introduced into the competent cells of DPAH6 obtained in Example 4. The method for preparing electrocompetent cells of DPAH6 is the same as that in Example 1 (3).
(4) Positive colonies of DPAH7 are constructed, and the construction method is the same as that in Example 1 (4).
(5) Plasmid elimination: The implementation method is the same as that in Example 1 (5), obtaining plasmid-free DPAH7 (DPAH6 derivative, yeeP::Trc-RBSApt2#82-*serAglyApanB*).
(6) The constructed DPAH7 production strain is subjected to shake-flask testing and detection using DPAH6 constructed in Example 2 as a control group. The OD₆₀₀ and the D-pantothenic acid content in the fermentation supernatant are shown in FIG. 4.

As can be seen from the figure, the overexpression of *serA* and *glyA* is controlled according to the intracellular glycine concentration. When glycine accumulates to a certain level, it folds to cover the ribosome binding site, repressing the translation of the *serA-glyA* gene cluster; when the intracellular glycine content is low, it exposes the ribosome binding site to enhance translation. In order to respond to the supply of methyl groups, we add one more copy of the *panB* gene after the gene cluster, obtaining strain H7. The D-pantothenic acid yield of DPAH7 was successfully increased to 6.52 g/L. Using the riboswitch to control the expression of *glyA* in real time achieves moderate enhancement of key genes of the serine pathway. This strategy can reasonably regulate the serine synthesis rate and maintain the intracellular glycine at a low level, successfully promoting D-pantothenic acid synthesis.

### Example 4: Construction of DPAH8 and Shake-Flask Fermentation

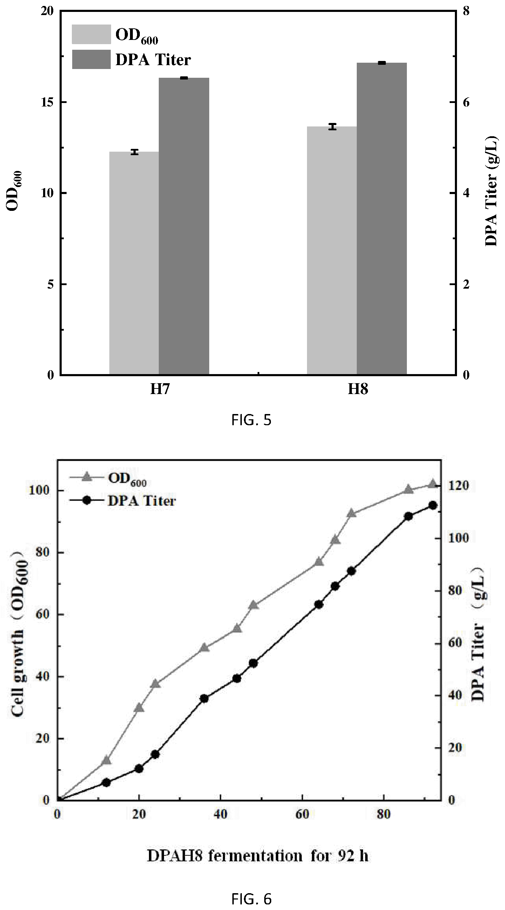

Using DPAH7 as the starting strain, CRISPR/Cas9 gene editing technology is employed to introduce a heterologous *alsS* gene into the genome of strain DPAH7 and perform codon optimization (nucleotide sequence shown as SEQ ID NO. 3), thereby obtaining DPAH8 (DPAH7 derivative, *gapC*::Trc-*alsS**).
(1) Construction of the pTarget-PAM-*alsS* plasmid: Using the pTarget plasmid (Addgene Plasmid #62226) as a template and pT-PAM-*alsS*-F/R as primers, PCR amplification is performed. After the PCR product is verified by nucleic acid gel electrophoresis, it is digested with Dpn I restriction enzyme at 37°C for 3 h, and then transformed into E.coli DH5α. After screening on spectinomycin plates and sequencing verification, the correct pTarget-PAM-*alsS* plasmid is obtained for subsequent ligation with Donor DNA.
(2) Construction of the pTD-Donor-*BsalsS* plasmid: First, using the E.coli W3110 genome as a template and *alsS*-up-F/R as primers, the upstream portion (F1) of the donor DNA is amplified, and using *alsS*-down-F/R as primers, the downstream portion (F2) of the donor DNA is amplified. Then, using the *Bacillus subtilis* 168 genome as a template and pTrc-*BsalsS*(2)-F/R as primers, the *BsalsS* gene fragment carrying the pTrc promoter (F3) is amplified. The PCR fragments F1, F2, and F3 are purified by gel extraction. The pTarget-*alsS* plasmid is digested with XbaI and PstI at 37°C for 8 h, and the DNA fragment is recovered using a Clean up kit. According to the instructions of ClonExpress^{®} (One step clone kit, Vazyme Biotech, Nanjing, China), the pTarget-*alsS* vector and fragments F1, F2, and F3 are ligated together, and the pTD-*BsalsS* plasmid is obtained through sequencing verification.
(3) The pCas plasmid (Addgene Plasmid #62225) is introduced into the competent cells of DPAH7 obtained in Example 5. The method for preparing electrocompetent cells of DPAH7 is the same as that in Example 1 (3).
(4) Positive colonies of DPAH8 are constructed, and the construction method is the same as that in Example 1 (4).
(5) Plasmid elimination: The implementation method is the same as that in Example 1 (5), obtaining plasmid-free DPAH8 (DPAH7 derivative, *gapC*::Trc-*alsS**).
(6) The constructed DPAH8 production strain is subjected to shake-flask testing and detection using DPAH7 constructed in Example 3 as a control group. The OD₆₀₀ and the D-pantothenic acid content in the fermentation supernatant are shown in FIG. 5.

Acetolactate synthase (*AlsS*) catalyzes the formation of α-acetolactate from two molecules of pyruvate, and acetolactate synthase is involved in the synthesis of valine, leucine, and isoleucine. As can be seen from the figure, by introducing a heterologous *alsS* gene into the genome and performing codon optimization, the expression efficiency of the gene in the host is improved, and the burden on the strain is relatively small. Without the addition of pyruvate, the shake-flask yield is promoted, and the D-pantothenic acid yield of strain DPAH8 is increased to 6.85 g/L.

### Example 5: Use of D-Pantothenic Acid-Producing Strain DPAH8 in Preparation of D-Pantothenic Acid by Microbial Fermentation

The fermentation is carried out in a 5 L fermenter (Shanghai Baoxing, BIOTECH-5BG), comprising the following steps:
(1) Seed culture: DPAH8 is inoculated from a plate into 10 mL of LB medium, and cultured overnight on a shaker at 37°C and 180 rpm. Thereafter, it is inoculated at a volume concentration of 1% into two bottles each containing 100 mL of LB medium as secondary seed liquid, and cultured for 7~12 h.
(2) Inoculation and fermentation: The volume of the fermentation medium in the 5 L fermenter is 2 L, sterilized at 115°C for 30 min. Conditions are 25~30°C, initial aeration rate of 3~6 L/min, initial stirring speed of 300~450 rpm, and pH adjustment with aqueous ammonia. A total of 200 mL of the two bottles of secondary seed liquid is transferred into the 5 L fermenter containing 2 L of antibiotic-free fermentation medium. At the same time, IPTG at a final concentration of 0.2 mM, VB₁ at a final concentration of 5 mg/L, VB₁₂ at a final concentration of 2 mg/L, and 5~10 mL of isoleucine at 10~40 g/L are added, and fermentation is started. During the fermentation process, dissolved oxygen is maintained at 10~30% using DO-cascade stirring, pH is maintained at 6.7~6.9 using aqueous ammonia as a neutralizing agent, the glucose concentration in the tank is controlled below 5 g/L through pH-stat feeding, and culture is carried out at 28~37°C for 3~4 days to obtain a fermentation broth. The fermentation broth refers to all substances within the fermenter.
(3) After diluting the fermentation supernatant 80-fold and subjecting the diluted sample to impurity removal treatment using an aqueous filter membrane, HPLC detection is performed according to Example 1. The OD₆₀₀ and the D-pantothenic acid content in the fermentation supernatant are shown in FIG. 6.

By regulating the glycine metabolic pathway, methyl cofactor supply, folate generation, and the serine synthesis pathway, the supply of one-carbon units is strengthened, allowing more metabolic flux to participate in the synthesis of D-pantothenic acid, thereby obtaining the D-pantothenic acid-producing strain DPAH8. As can be seen from the figure, the engineered strain DPAH8 fermented in a 5 L fermenter for 92 hours yields a D-pantothenic acid production increased to 112.62 g/L, greatly improving the synthesis of D-pantothenic acid.

Wherein, the fermentation medium has the following composition: glucose 20 g/L, ammonium sulfate 16 g/L, anhydrous betaine 2 g/L, yeast extract 2 g/L, potassium dihydrogen phosphate 2 g/L, anhydrous magnesium sulfate 0.5 g/L, β-alanine 1.5 g/L, 1 mL/L trace element solution, with deionized water as solvent, and natural pH. The trace element solution has the following composition: 10 g/L CuCl₂, 10 g/L FeSO₄·7H₂O, 10 g/L ZnSO₄·7H₂O, 0.2 g/L CuSO₄, 0.02 g/L NiCl₂·7H₂O, with deionized water as solvent.

Wherein, the feed medium has the following composition: glucose 500 g/L, ammonium sulfate 10 g/L, anhydrous betaine 4 g/L, yeast extract 2 g/L, potassium dihydrogen phosphate 14 g/L, anhydrous magnesium sulfate 8 g/L, β-alanine 60 g/L, 2 mL/L trace element solution, with deionized water as solvent, and natural pH.

The primer sequence information used in Examples 1 to 4 is shown in Table 2.

**Table 2: Primer Sequences**

| Primer | Sequence (5'-3') |
|---|---|
| *gcvT-PAM-*F | TAATACTAGTTCAGCCCCGCTTATTCAATGGTTTTAGAGCTAGAAATAGC |
| *gcvT-PAM-*R | GCTCTAAAACCATTGAATAAGCGGGGCTGAACTAGTATTATACCTAGGAC |
| *gcv*T-up-F | CTTTTTTTGAATTCTCTAGAGACATCGGGCA GTACATTT |
| *gcvT-up-R* | TTCCACACATTATACGAGCCGGATGATTAATTGTCAAAAAAATGCAAAAA TCGCGACAG |
| gcvT-down-F | GGCTCGTATAATGTGTGGAATTTCACACAGGAAACAGACCATGGCACAA CAGACTCCTT |
| gcvT-down-R | GTGAAACATTATACGAGCCGGATGATTAATTGTCAAACTGGTAAAGTCCT GACAATACT |
| *pntAB-PAM* -F | TAATACTAGTTCGTACATGAGCAGCTTGTGGTTTTAGAGCTAGAAATAGC |
| *pntAB-PAM* -R | GCTCTAAAACCACAAGCTGCTCATGTACGAACTAGTATTATACCTAGGAC |
| *pntAB-up-F* | ATTCTCTAGAGTCGACCTGCTCCGGTGTCAGTTCTGTTGC |
| *pntAB-up-R* | ATTCCACACATTATACGAGCCGGATGATTAATTGTCAAACGCCCTATAACG TACTG |
| *pntAB-down* -F | GCTCGTATAATGTGTGGAATTTCACACAGGAAACAGACCATGCGAATTG GCATACCAAG |
| *pntAB-down* -R | GGGTAATAGATCTAAGCTTCTTTGCCGGTGGCACTTTCC |
| *folP-PAM-F* | TAATACTAGTATCCGCTCCCTTTCCGAACCG TTTAGAGCTAGAAATAGC |
| *folP-PAM-R* | GCTCTAAAACGGTTCGGAAAGGGAGCGGATACTAGTATTATACCTAGGAC |
| *folP-Fl* | TCTCTAGAGTCGACCTGCAGTAATCCCGGTG GCGGTGT |
| *folP*-R1 | GCTGTTGACAATCAACTTTATT |
| *folP-F2* | TAAAGTTGATTGTCAACAGCTTGACAATTAA TCATCCGGCTC |
| *folP-R2* | AATGCGTAAAGATTCAGCATTTACTCATAGC GTTTGTTTTCCT |
| *folP-F3* | ATGCTGAATCTTTACGCATTTCTC |
| *folP-R3* | GAGCTGCACATGAACTCGAGGTGCCACGCC CGCAGACG |
| *yeeP-PAM-*F | TAATACTAGTGAATATTCGCGAAAAAACGGG TTTTAGAGCTAGAAATAGC |
| *yeeP-PAM-*R | GCTCTAAAACCCGTTTTTTCGCGAATATTCAC TAGTATTATACCTAGGAC |
| *yeeP-up-F* | ATTCTCTAGAGTCGACCTGCCGACACCCGGA GACAACGGC |
| *yeeP-down-*R | GGGTAATAGATCTAAGCTTCTCACAATCTGG CGACCGTTTTT |
| *yeeP-XF* | ACCTGTCGTGACTGATGCCC |
| *Trc-yeeP-F* | GACATCAGCAGGTGCTGTTTTTGACAATTAA TCATCCGGCTC |
| Apt2-82-R1 | ACTTTCTCCTTCGGTGCTCATCAAGAGTCTC TCCAAGGTTCCACACATTATACGAGCCG |
| Apt2-82-R2 | AGAGTTTTGCCGTACGACTTTCTCCTTCGGT GCTCAT |
| Apt2-82-FB | AAGTCGTACGGCAAAACTCTCAGGTAAAAG GACAGGGAAAACCCTTCTCGAAC |
| *82-serA-F* | CTCGAACTAAGGAGCAAACTATGGCAAAGG TATCGCTGGAG |
| 82-XR | AGTTTGCTCCTTAGTTCGAGAAG |
| glyA-s-F | GCGCCCGTCTGCTGTACTAACCTTGGAGAGA CTCTTGATGAG |
| glyA-s-R | AGTCACATTGGTGGGGGCAATTATGCGTAAA CCGGGTAACG |
| *yeeP-XR* | AAACAGCACCTGCTGATGTCC |
| P1-1 | CCAATGTGACTGATAAAATGCCAGACATTTC ACACAGGAAACAGACC |
| P1-2 | GGCATTTTATCAGTCACATTGGTGGGGGCAATTAGTACAGCAGACGGGC GC |
| *panB-yeeP-*R | GGGCATCAGTCACGACAGGTTTATTTTCCCC CGTACAAGC |
| *glyA-F* | ACACAGGAAACAGACCATGGAATTCATGTTAAA GCGTGAAATGAACAT |
| *glyA-R* | CCGCCAAAACAGCCAAGCTTTTATGCGTAAACC GGGTAACG |
| *folP-F* | TTTCACACAGGAAACAGACCATGCCATCACTCA GTAAAGAAGCG |
| *folP-R* | CCGCCAAAACAGCCAAGCTTTCAGTTGTGATGA CGCACAGCGC |
| *folC-F* | TTTCACACAGGAAACAGACCATGATTATCAAAC GCACTCCTC |
| *folC-R* | CCGCCAAAACAGCCAAGCTTTTACTTGCCACCG CTTCTCC |
| *folM-F* | TTTCACACAGGAAACAGACCATGGGTAAAACCC AGCCCTTG |
| *folM-R* | CCGCCAAAACAGCCAAGCTTTTAACGCAGATGA CGACCGC |
| *folA-F* | TTTCACACAGGAAACAGACCATGATCAGTCTGA TTGCGGCG |
| *folA-R* | CCGCCAAAACAGCCAAGCTTTTACCGCCGCTCC AGAATCT |
| *alsS-R* | GCTATTTCTAGCTCTAAAACCCGTGGGCGATTTA CAACTGACTAGTATTA |
| *alsS-1* | GCTTTTTTTGAATTCTCTAGAAAGGATATTATTAT CCGCCGACATTGC |
| *alsS-3* | CCTGTGCGCGTTACAGTGCTTTGGTTTTCATCAG TTCACC |
| *alsS-2* | CAAAGCACTGTAACGCGCACAGGCGGAG |
| *alsS-RBS-F* | GGTCTGTTTCCTGTGTGAAACAATTCCACACATTATACGAGCCGGATGAT TAAT |
| *alsS-RBS-R* | TTTCACACAGGAAACAGACCATGACCAAAGCCA CCAAAGAAC |
| *alsS-6* | TAATAGATCTAAGCTTCTGCAGATCGGCGGGGTT GGGTAT |

The embodiments described above are merely descriptions of preferred embodiments of the present invention, and are not intended to limit the scope of the present invention. Without departing from the design spirit of the present invention, various modifications and improvements made to the technical solutions of the present invention by a person of ordinary skill in the art shall fall within the protection scope of the present invention.

## Claims

1. A method for constructing a genetically engineered bacterium for high-yield production of D-pantothenic acid, **characterized in that** the method comprises: using CRISPR-Cas9-mediated gene editing technology, in the genome of a chassis bacterium: overexpressing the *gcvTHP* gene; enhancing the *folP* gene; integrating a *serA* and *glyA* gene cluster and adding one copy of the *panB* gene after the *serAglyA* gene cluster, wherein the *serAglyA* gene cluster is regulated by a dynamic regulatory element; and introducing a heterologous codon-optimized *alsS* gene, thereby obtaining a genetically engineered bacterium for high-yield production of D-pantothenic acid.

2. The method according to claim 1, **characterized in that** the method for enhancing the *folP* gene comprises: adding one copy of the *folP* gene at the *lafU* pseudogene locus.

3. The method according to claim 1, **characterized in that** the dynamic regulatory element is a riboswitch Apt2#82.

4. The method according to claim 1, **characterized in that** the *panB* gene is regulated by the PpanB promoter.

5. The method according to claim 1, **characterized in that** the *alsS* gene is derived from *Bacillus subtilis* and is codon-optimized.

6. The method according to claim 1, **characterized in that** the chassis bacterium is: E.coli W3110, Trc-*EcilvD**/ *BspanBA**/ *CgpanC**/ *alsS**/ *BspanB**/ *nac^{GTG}*/ ΔP_{345ptsH}/ *gltA^{GTG}*/ *gltA^{TTG}*/ Trc-*PpfkB*.

7. The method according to claim 1, **characterized in that** the nucleotide sequences of the *gcvTHP* gene, the *folP* gene, and the *alsS* gene regulated by the Ptrc promoter are shown as SEQ ID NO. 1, SEQ ID NO. 2, and SEQ ID NO. 3, respectively; the sequence of the *serAglyA* gene cluster regulated by the Apt2#82 riboswitch is shown as SEQ ID NO. 4; the nucleotide sequence of the *panB* gene regulated by the PpanB promoter is shown as SEQ ID NO. 5.

8. A genetically engineered bacterium for high-yield production of D-pantothenic acid constructed by the method according to any one of claims 1 to 7.

9. Use of the genetically engineered bacterium for high-yield production of D-pantothenic acid according to claim 8 in the preparation of D-pantothenic acid by microbial fermentation.

10. The use according to claim 9, **characterized in that** the method of the use comprises: inoculating the genetically engineered bacterium for high-yield production of D-pantothenic acid into a fermentation medium, performing fed-batch fermentation culture at 28~37°C, 300~450 rpm, pH 6.7~6.9, and dissolved oxygen of 10~30% for 72~96 h, and after fermentation is completed, separating and purifying to obtain the D-pantothenic acid.
